# EUROPEAN PATENT APPLICATION

(11) **EP 4 678 112 A1**
(43) Date of publication of application: **14.01.2026**
(21) Application number: 24187302.5
(22) Date of filing: 09.07.2024
(51) Int. Cl.: A61B 6/06, A61B 6/08, A61B 6/00, A61B 6/58

(54) **AUTO-COLLIMATION VIA LIGHT FIELD**

(71) Applicant: Koninklijke Philips N.V., 5656 AG Eindhoven (NL)
(72) Inventor: GOOSSEN, André, Eindhoven (NL); BYSTROV, Daniel, Eindhoven (NL); YOUNG, Stewart Matthew, 5656AG Eindhoven (NL); SENEGAS, Julien Thomas, Eindhoven (NL); KROENKE-HILLE, Sven, Eindhoven (NL); VON BERG, Jens, Eindhoven (NL)
(74) Representative: Philips Intellectual Property & Standards

(57) **Abstract**

An apparatus and a method are provided for determining a collimation configuration in an imaging system. Image data is accessed that is acquired by a two-dimensional (2D) camera of a camera system. The 2D camera has a field of view to capture a scene containing a subject. The image data is obtained prior to the imaging examination of the subject. A machine learning algorithm is applied to the image data to determine a geometry parameter of a target light field of collimation that is expected to be projected onto the subject. The geometry parameter of the light field of collimation is provided for guiding or controlling the adjustment of the geometry of the collimator.

## Description

### FIELD OF THE INVENTION

The present invention relates to an apparatus and a method for determining a collimation configuration in an imaging system, and to a computer readable medium comprising instructions for causing a processor to perform the methods.

### BACKGROUND OF THE INVENTION

A light field that is aligned with the X-ray beam is used to preview collimation and perform an ideal exam according to guidelines and an As Low as Reasonably Achievable(ALARA) principle. The light field is projected onto the patient and is visible by the radiographer but also external sensors.

Diagnostic X-ray systems that automatically configure their settings prior to acquisition of an exam, and adapt to patient properties using dedicated AI approaches, have become reality and vendors are increasingly adopting such techniques. AutoCollimation for the DigitalDiagnost system, scheduled for imminent release, features a landmark detector which evaluates depth images of a patient's thorax in order to detect internal landmarks, i.e., locations indicating the extent of the lung parenchyma, which is trained using chest X-ray images acquire simultaneously. This technique is robust and clinically proven and a key differentiator for the workflow in chest X-ray exams.

### SUMMARY OF THE INVENTION

There may be a need to provide a system and method for automatic collimation which does not require any interaction with the X-ray system and thus can be used to retrofit many X-ray systems with automatic collimation.

The object of the present invention is solved by the subject-matter of the appended independent claims, wherein further embodiments are incorporated in the dependent claims.

According to a first aspect of the present invention, there is provided an apparatus for determining a collimation configuration in an imaging system. The apparatus comprises:
- a camera data interface configured to access image data that is acquired by a two-dimensional, 2D, camera of a camera system, wherein the 2D camera has a field of view to capture a scene containing a subject, wherein the image data is obtained prior to an imaging examination of the subject;
- a memory comprising instruction data representing a set of instructions;
- a processor configured to communicate with the camera data interface and the memory and to execute the set of instructions, wherein the set of instructions, when executed by the processor, cause the processor to apply a machine learning algorithm to the image data to determine a geometry parameter of a target light field of collimation that is expected to be projected onto the subject, wherein:
- the machine learning algorithm is represented by algorithm data which is stored in the memory and accessed by the processor, and
- the machine learning algorithm has been trained using a training dataset comprising a plurality of training data examples, wherein the plurality of training examples comprises one or more of:
   - a training data example comprising (i) image data of a scene comprising a subject with a light field of collimation projected onto said subject, wherein the image data was obtained during a previous imaging procedure for the same or another subject, and (ii) a geometry parameter of the collimation derived from the image data; and
   - a training data example comprising (i) an X-ray image that was acquired during a previous imaging procedure of the same or another subject, said X-ray image showing a subject's exterior, and (ii) a geometry parameter of the collimation derived from the X-ray image; and
- an output interface configured to output the geometry parameter of collimation. Accordingly, the apparatus as described herein is provide for automatic collimation which does not require any interaction with the X-ray system and thus can be used to retrofit many X-ray systems with automatic collimation.

According to an embodiment of the present invention, the image data shows a current light field of the collimation that is projected onto the subject. The set of instructions, when executed by the processor, cause the processor to:
- determine a deviation between a geometry parameter of the current light field of the collimation and the geometry parameter of the target light field of the collimation; and
- if the deviation exceeds a threshold, generate a collimation adjustment guidance indicative of the determined deviation and/or generate a control signal, which is used for controlling a collimator of the imaging system to adjust a collimator setting to reduce the deviation.

Accordingly, the camera system may acquire 2D frames of the scene containing the subject. The machine-learning algorithm is applied to predict the geometry parameter of the target light field of the collimation, such as the size and the position of the target light field. The deviation in size and position of the light field between the current light field and the target light field may be determined and indicated using e.g., a screen with live image and the target light field, a visual instruction such as LEDs, and(or an audible command. Alternatively, a control signal may be generated. The control signal may be used to fit motor to control knobs or handles to adjust the collimator. Alternatively or additionally, the control signal may inject system commands to control the collimator.

According to an embodiment of the present invention, the set of instructions, when executed by the processor, cause the processor to continuously monitor the current light field of collimation that is projected onto the subject, and determine the deviation between the geometry parameter of the current light field of the collimation and the geometry parameter of the target light field of the collimation, until the deviation is less than the threshold.

Accordingly, the above steps may be repeated to continuously determine the deviation between the current light field and the target light field. The apparatus 10 may control the adjustment of the collimator via a feedback loop based on the deviation.

According to an embodiment of the present invention, the collimation adjustment guidance comprises one or more of the following:
- a display showing the target light field of the collimation that is overlaid on the current light field of the collimation projected onto the subject;
- a visual instruction; and
- an audio command.

According to an embodiment of the present invention, the control signal comprises one or more of:
- a control signal configured to control a motor to control a knob to adjust the collimator of the imaging system; and
- a control signal configured to inject a system command to control the collimator of the imaging system.

According to an embodiment of the present invention, at least one training data example further comprises a score indicative of a collimation quality of an X-ray image acquired during the previous imaging procedure.

Accordingly, the score may be used as an additional input to indicate a collimation quality of an X-ray image. This may be used to adjust the geometry parameter of the target light field of collimation.

According to an embodiment of the present invention, the score comprises a user input score, or a score generated based on an analysis of the X-ray image.

According to an embodiment of the present invention, the 2D camera comprises an RGB camera.

According to an embodiment of the present invention, the camera data interface is further configured to access image data acquired by a three-dimensional, 3D, camera of the camera system. At least one training data example further comprises image data acquired by a 3D camera during the previous imaging procedure. The set of instructions, when executed by the processor, cause the processor to use the image data acquired by the 3D camera of the camera system as additional input to the machine learning algorithm.

In this embodiment, the 2D image data may be used to detect the current light field of the collimation, whereas the data from any or all the other sensors are used to predict the optimal light field. This embodiment may require that the different sensor images of the camera system are registered spatially, which is typically the case. Hence, the parameters of the current light field, during training and inference, can be transformed from the 2D data to the data of the other sensors. The machine learning algorithm may be trained using features of any of the sensor data.

According to an embodiment of the present invention, the apparatus further comprises subject data interface configured to access the non-image subject data from an electronic health record of the subject. At least one training data example further comprises non-image subject data. The set of instructions, when executed by the processor, cause the processor to use the non-image subject data as additional input to the machine learning algorithm.

Accordingly, the non-image data of the subject may be used to tailor the target light field to suit the subject.

According to an embodiment of the present invention, the non-image subject data of the subject comprises at least one of:
- a weight of the subject;
- an age of the subject;
- a gender of the subject;
- a quantification of a fitness level of the subject;
- a disease diagnosis associated with the subject;
- a medication record associated with the subject; and
- a vital parameter record associated with the subject

According to an embodiment of the present invention, the geometry parameter of the target light field of collimation comprises a size of the light field of the collimation and a position of the light field of the collimation with respect to the subject.

In some examples, the light field of the collimation may have a rectangular or square shape.

In some examples, the light field of the collimation may have a circular shape.

In some further examples, the light field of the collimation may have a hexagonal shape.

According to an embodiment of the present invention, the machine learning algorithm comprises at least one of: a convolutional neural network (CNN), a transformer, a generalized Hough transform, and an edge detection algorithm.

According to a second aspect of the present invention, there is provided a method for determining a collimation configuration in an imaging system, the method comprising:
- accessing image data that is acquired by a two-dimensional (2D) camera of a camera system, wherein the 2D camera has a field of view to capture a scene containing a subject, wherein the image data is obtained prior to the imaging examination of the subject;
- applying a machine learning algorithm to the image data to determine a geometry parameter of a target light field of collimation that is expected to be projected onto the subject, wherein:
   the machine learning algorithm has been trained using a training dataset comprising a plurality of training data examples, wherein the plurality of training examples comprises one or more of:
   - a training data example comprising (i) image data of a scene comprising a subject with a light field of collimation projected onto said subject and an X-ray detector, wherein the image data was obtained during a previous imaging procedure for the same or another subject, and (ii) a geometry parameter of the light field of collimation derived from the image data; and
   - a training data example comprising (i) an X-ray image retrieved from a picture archiving and communication system, PACS, said X-ray image showing a subject's exterior, and (ii) a geometry parameter of the light field of collimation derived from the X-ray image, and meta-data comprising a collimation setting and geometry data of an imaging system for acquiring the retrieved X-ray image; and
- outputting the geometry parameter of the light field of collimation, which is usable for setting up collimation for the subject.

According to a further aspect of the present invention, there is provided a computer readable medium comprising transitory or non-transitory data representing instructions arranged to cause a processor system to perform the method according to the second aspect.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 illustrates an exemplary X-ray imaging system for imaging a subject.
Fig. 2 illustrates an exemplary machine learning algorithm.
Fig. 3 illustrates an exemplary training pair.
Fig. 4 illustrates a further exemplary training pair.
Fig. 5 illustrates an exemplary deployment phase of the machine learning algorithm.
Fig. 6 illustrates a further exemplary X-ray imaging system.
Fig. 7 illustrates a flow chart depicting an exemplary method for determining a collimation configuration in an imaging system.

### DETAILED DESCRIPTION OF EMBODIMENTS

The following description presents exemplary apparatus, systems, and methods for determining a collimation configuration in an imaging system. Particularly, embodiments illustrated hereafter disclose apparatus, imaging systems, and methods that do not require system integration and can be used to upgrade an installed base of X-ray systems using the collimator's light field and dedicated end-to-end machine learning algorithms to set-up a control system for automatically providing feedback on collimation or setting up the collimation for the radiographer for the current patient. The apparatus, imaging systems, and methods may rely on an additional camera and processor and can either learn from skilled operators or the medical images as ground truth. In some example, in training a light field of the collimator of the diagnostic system is segmented, and its size and position may be used to train an end to-end network predicting these size and position parameters, and during inference the predicted size and position is indicated and the actual light field (e.g., segmented from the live video) of the diagnostic X-ray system is adjusted until it coincides with the predicted light field. In some examples, coinciding of the optimum and actual light field may be achieved by using a video screen with live image and optimum overlay or using visual instructions such as LEDs or using audible commands.

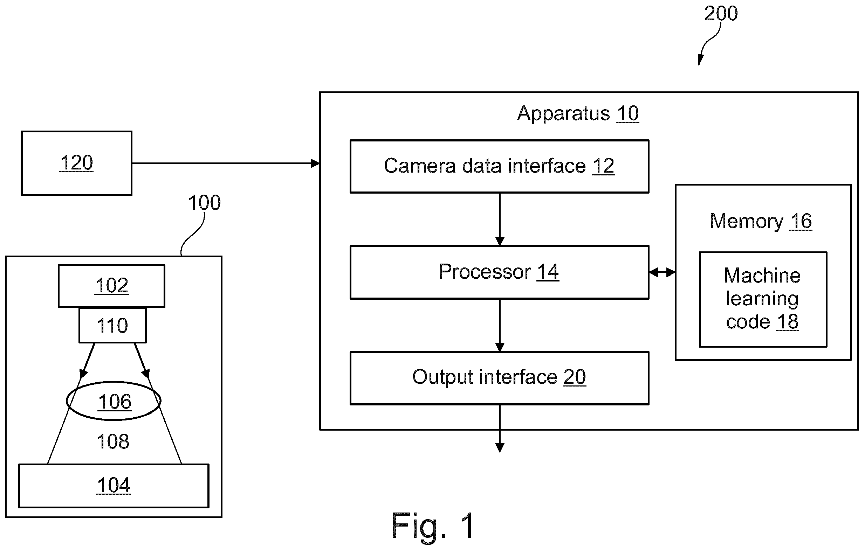

It will be appreciated that use of the present disclosure in various X-ray imaging applications and systems is contemplated. Some of these systems may include a CT scanner, a positron emission tomography CT (PET-CT) scanner, a single and/or multiple source imaging system, a single and/or multiple detector imaging system, a single photon emission computed tomography-CT (SPECT-ST) scanner, an X-ray tomosynthesis system, a magnetic resonance-X-ray (MRX) scanner, an MR-CT scanner, and/or any imaging system that uses an X-ray tube. An exemplary environment that is suitable for practicing various implementations of the present disclosure will be discussed in the following sections with reference to Fig. 1.

Fig. 1 illustrates an exemplary X-ray imaging system 200 for imaging a subject, such as a medical patient.

The X-ray imaging system 200 comprises an X-ray imaging device 100 configured to acquire image data of a subject, such as a medical patient. In some examples, the X-ray imaging device 100 may be configured to acquire projection data using a single X-ray source spectrum. In some other examples, the X-ray imaging device 100 may be configured to acquire projection data, for example, at two or more X-ray energy levels for further display and/or analysis. In some further examples, the X-ray imaging device 100 may be a CT system that may include a gantry (not shown). The X-ray imaging device 100 comprises an X-ray tube 102 and an X-ray detector 104 configured to acquire X-ray image data of a subject 106. The X-ray tube 102 and the X-ray detector 104 are separated by an examination region 108 for performing an X-ray imaging operation on the subject 106 when the subject 106 is located within the examination region 108. The solid lines extending between the X-ray tube 102 and the X-ray detector 104 indicate the volumetric extent of the overlap between the X-ray beam emitted by the X-ray tube 102, and the X-ray radiation-sensitive region of the X-ray detector 104, within which the X-ray image data may be generated. The volumetric extent of this overlap defines the examination region 108.

The X-ray tube 102 may be supported by an X-ray tube support device. In some examples, the X-ray tube support device may be a celling support device. The celling support device may comprise two perpendicular sets of ceiling-mounted rails, which allow for both longitudinal and transverse travel of the X-ray tube. In some other examples, the X-ray tube support device may be a floor to ceiling support device. The floor to ceiling support device may have a single column with rollers at each end, one attached to a celling mounted rail and the other attached to a floor mounted rail. The tube slides up and down the column as the column rotates. The X-ray tube support device may be a C-arm support device, which is ceiling mounted and provides for flexible tube positioning, and the X-ray detector is attached to the other end of the C-arm from eh X-ray tube. In some further examples, the X-ray tube 102 and the X-ray detector 104 may be positioned on the opposite sites of the gantry of a CT scanner. Alternative arrangements, mounting arrangements, and positions of the X-ray tube 102 and the X-ray detector 104 may also be used.

The X-ray imaging device 100 further comprises a collimator 110 configured to collimate the X-ray beams based on specific imaging and/or examination requirements using one or more collimating regions, for example defined using lead or tungsten shutters.

A camera system 120 is arranged, which may be mounted to the X-ray imaging device 100, e.g., in the tube head, or placed detached from the system, e.g., ceiling mounted. The camera system 120 comprises a 2D camera that has a field of view to capture a scene containing the subject 106 and an X-ray detector 104. In some examples, the 2D camera 120 may have a field of view that at least partly overlaps the field of view of the X-ray imaging device. The 2D camera 120 is configured to acquire a sequence of 2D images of the subject 106 by exposure of non-ionizing radiation, e.g., visible light, infrared light, etc. For example, the 2D camera 120 may be an RGB camera. The sequence of 2D images captures the subject's exterior of the subject 106 or at least the torso part of the subject 106. While Fig. 1 may show one camera by way of example, it will be appreciated that in some other implementations,

The sequence of 2D images is then fed into the apparatus 10 for determining a collimation configuration in the X-ray imaging device 100 via a camera data interface 12. The camera data interface may be implemented as an Ethernet interface, a USB (TM) interface, a wireless interface such as a WiFi (TM) or Bluetooth (TM) or any comparable data transfer interface enabling data transfer between the 2D camera 120 and the apparatus 10.

The apparatus 10 further comprises a processor 14 and a memory 16. The processor 14 is configured to communicate with the camera data interface 12 and the memory 16 and to execute the set of instructions. The set of instructions, when executed by the processor, cause the processor to apply a machine learning algorithm to the image data to determine a geometry parameter of collimation.

The processor 14 may perform a machine learning algorithm by executing machine learning code 18 embodying the algorithm. In some examples, the processor 16 may access and execute code for potentially multiple different machine learning algorithms, and may even in some cases, perform multiple algorithms in parallel. The processor 14 may include any processor or processing device, such as a microprocessor, an embedded processor, a digital signal processor (DSP), a network processor, a handheld processor, an application processor, a co-processor, a system on a chip (SOC), or other device to execute code 18. The machine learning code 18 embodying a machine learning algorithm may be stored in the memory 16, which may be local or remote to the processor 14. The memory 16 may be implemented as one or more of shared memory, system memory, local processor memory, cache memory, etc., and may be embodied as software, firmware, or a combination of software and firmware.

The machine learning algorithm has been trained using a training dataset comprising a plurality of training data examples. In some examples, the plurality of training examples may comprise a training data example comprising (i) image data of a scene comprising a subject with a light field of collimation projected onto said subject and an X-ray detector. The image data was obtained during a previous imaging procedure for the same or another subject, and (ii) a geometry parameter of the collimation derived from the image data. Alternatively or additionally, the plurality of training examples may comprise a training data example comprising (i) an X-ray image retrieved from a picture archiving and communication system, PACS, said X-ray image showing a subject's exterior, and (ii) a geometry parameter of the collimation derived from the X-ray image, and meta-data comprising a collimation setting and geometry data of an imaging system for acquiring the retrieved X-ray image. The apparatus 10 further comprises an output interface 20, which is configured to output the geometry parameter of collimation.

The output interface 20 may be implemented as an Ethernet interface, a USB (TM) interface, a wireless interface such as a WiFi (TM) or Bluetooth (TM) or any comparable data transfer interface enabling data transfer between the output interface and another device.

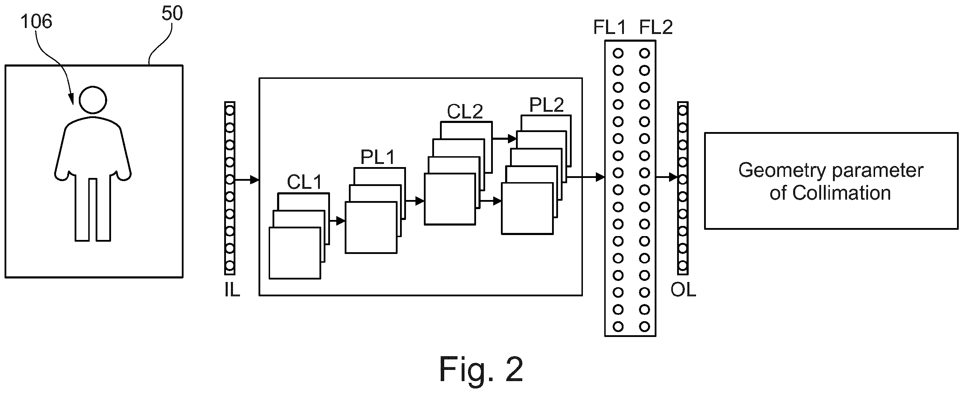

Reference is now made to Fig. 2, where a neural-network model is shown as an exemplary data-driven model. However, other machine learning techniques such as support vector machines, decision trees or other may be used instead of neural networks. Having said that, neural networks, in particular convolutional networks, have been found to be of particular benefit especially in relation to image data.

Reference is now made to Fig. 2, where a neural-network model is shown as an exemplary machine learning algorithm. However, other machine learning techniques such as a transformer, a generalized Hough transform, an edge detection algorithm or other may be used instead of neural networks. Having said that, neural networks, in particular convolutional networks, have been found to be of particular benefit especially in relation to image data.

Specifically, Fig. 2 is a schematic diagram of a convolutional neural-network (CNN). A fully configured NN as obtained after training may be thought as representation of an approximation of a latent mapping between two spaces, the images and the space of a geometry parameter of collimation. These spaces can be represented as points in a potentially high dimensional space, such as an image being a matrix of *N × N,* with *N* being the number of pixels. The geometry parameter of collimation may be similarly encoded as vectors, matrices or tensors. The learning task may be one or more of classification and/or regression. In some examples, the input space of images may include 4D matrices to represent a time series of matrices, and hence a video sequence.

A suitable trained machine-learning algorithm or component attempts to approximate this mapping. The approximation may be achieved in a learning or training process where parameters, itself forming a high dimensional space, are adjusted in an optimization scheme based on training data.

In yet more detail, the machine-learning component may be realized as NN, in particular a CNN. With continued reference to Fig. 2, this shows in more detail a CNN architecture as envisaged herein in embodiments.

The CNN is operable in two modes: "training mode/phase" and "deployment mode/phase". In training mode, an initial model of the CNN is trained based on a set of training data to produce a trained CNN model. In deployment mode, the pre-trained CNN model is fed with non-training, new data, to operate during normal use. The training mode may be a one-off operation or this is continued in repeated training phases to enhance performance. All that has been said so far in relation to the two modes is applicable to any kind of machine learning algorithms and is not restricted to CNNs or, for that matter, NNs.

The CNN comprises a set of interconnected nodes organized in layers. The CNN includes an output layer OL and an input layer IL. The input layer IL may be a matrix whose size (rows and columns) matches that of the training input image. The output layer OL may be a vector or matrix with size matching the size chosen for the geometry parameter of the collimation.

The CNN has preferably a deep learning architecture, that is, in between the OL and IL there is at least one, preferably two or more, hidden layers. Hidden layers may include one or more convolutional layers CL1, CL2 ("CL") and/or one or more pooling layers PL1, PL2 ("PL") and/or one or more fully connected layer FL1, FL2 ("FL"). CLs are not fully connected and/or connections from CL to a next layer may vary but are in generally fixed in FLs.

Nodes are associated with numbers, called "weights", which represent how the node responds to input from earlier nodes in a preceding layer.

The set of all weights defines a configuration of the CNN. In the learning phase, an initial configuration is adjusted based on the training data using a learning algorithm such as forward-backward ("FB")-propagation or other optimization schemes, or other gradient descent methods. Gradients are taken with respect of the parameters of the objective function.

The training mode is preferably supervised, that is, is based on annotated training data. For each pair, one item is the training input data and the other item is target training data known a priori to be correctly associated with its training input data item. This association defines the annotation. The annotation may be provided by a human expert and/or may be generated automatically using an algorithm to analyze the the training input data. The training pair includes imagery as training input data, and associated with each training image, is target of label for geometry parameter of collimation.

As noted above, in the present disclosure, annotated training data may include pairs or training data items as described below.

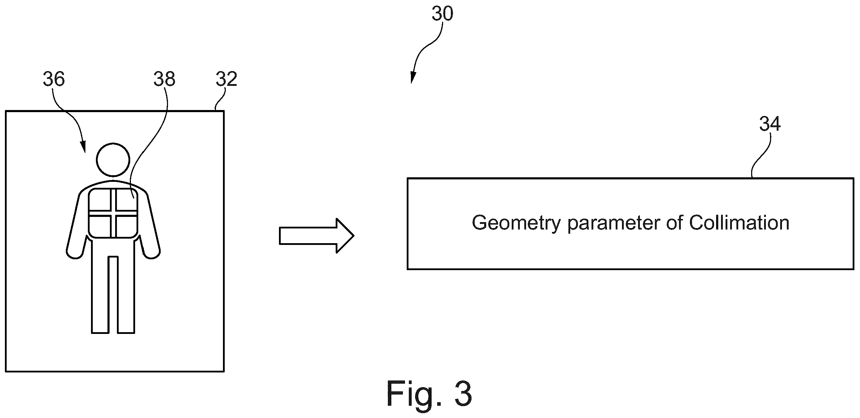

In some examples, the annotated training data may comprise a training data example that comprises (i) image data of a scene comprising a subject with a light field of collimation projected onto said subject and an X-ray detector. The image data was obtained during a previous imaging procedure for the same or another subject, and (ii) a geometry parameter of the collimation derived from the image data. For example, Fig. 3 illustrates an exemplary training pair 30 that comprises image data 32 as training input data, and a target of label 34 for geometry parameter of collimation. The image data 32 was obtained during a previous imaging procedure for the same or another subject. For example, the image data 32 was obtained during collimation. As shown in Fig. 3, the image data captured the scene comprising a subject 36 with a light field of collimation 38 projected onto said subject 36. In some examples, the subject 36 may be the same as the subject 106 shown in Fig. 1. In some examples, the subject 36 may be different from the subject 106 shown in Fig. 1. During collimation, the light field of collimation 38 may be detected, and the geometry parameter of collimation 36 during the actual X-ray acquisition are stored as the target of label 34. The geometry parameter of collimation 36 may comprise a size of the light field of the collimation 38 and a position of the light field of the collimation 38 with respect to the subject 36. In some implementations, image segmentation may be used to detect the light field of the collimation 38 in the image data 32. In some implementations, the light field of collimation 38 may be not segmented, but a bounding box or polygon is directly regressed using approaches like YOLO-based object detection as described in the article "You Only Look Once: Unified, Real -Time Object Detection " by J. Redmon et al., 2015. YOLO was designed to predict bounding boxes and class probabilities (e.g., x, y, width, height, probability) for an object of interest in an RGB image. Optionally, in addition to the image data, the training input data may further comprise non-image subject data from an electronic health record of the subject 36. The non-image subject data of the subject may comprise one or more of a weight of the subject, an age of the subject, a gender of the subject, a quantification of a fitness level of the subject, a disease diagnosis associated with the subject, a medication record associated with the subject, and a vital parameter record associated with the subject. The non-image subject data may be used to improve the prediction of the geometry parameter of the collimation that is tailored to a particular subject.

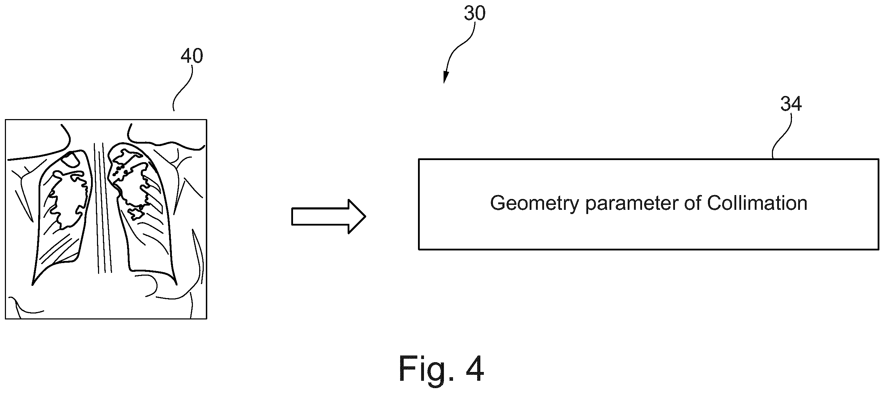

In some examples, the annotated training data may comprise a training data example that comprises (i) an X-ray image that was acquired during a previous imaging procedure of the same or another subject, said X-ray image showing a subject's exterior, and (ii) a geometry parameter of the collimation derived from the X-ray image. As an example, the X-ray image may be an image that is stored in a picture archiving and communication system, PACS. The machine learning algorithm may be trained with access to the PACS. For example, Fig. 4 illustrates a further exemplary training pair 30 that comprises X-ray image data 40 as training input data. The X-ray image data 40 shows a subject's exterior. The geometry parameter of collimation may be derived from the X-ray image data by processing the X-ray image data to detect the collimator blades. The information obtained from the detection of the collimator blades is then used to determine the area enclosed by the blades. The area enclosed by the collimator blades is referred to as the shutter area. This may be done by using an Auto Schutter process, which may comprise the following two steps: (1) the use of edge detection algorithms, such as the Hough transform, to detect the potential edges of the collimator blades in the image, which appear as straight lines; and (2) the selection of desirable edges. Once the shutter area is determined, the size and position of the shutter area with respect to the patient's exterior may be stored as the geometry parameter of the collimation 34. Optionally, the X-ray image data 40 may comprise meta-data on collimation, such as the shutter-cropped region. In this case, the shutter area may be determined by taking into account the meta-data on collimation. In some implementations, in addition to the X-ray image data 40, the training input data may further comprise non-image subject data from an electronic health record of the subject 36. The non-image subject data of the subject may comprise one or more of a weight of the subject, an age of the subject, a gender of the subject, a quantification of a fitness level of the subject, a disease diagnosis associated with the subject, a medication record associated with the subject, and a vital parameter record associated with the subject. The non-image subject data may be used to improve the prediction of the geometry parameter of the collimation that is tailored to a particular subject. In some implementations, in addition to the geometry parameter of collimation, the target of labels may further comprise a score indicative of a collimation quality of the X-ray image data. In some examples, the score may comprise a user input score. Fin one example, the score may be provided by an experienced radiographer as a label. In another example, the score may be generated based on an analysis of the X-ray image data 40. As a further option, the training input data may further com

In some examples, the annotated training data may comprise both training data examples shown in Figs. 3 and 4.

In training mode, preferably multiple such pairs are applied to the input layer to propagate through the CNN until an output emerges at OL. Initially, the output is in general different from the target. During the optimization, the initial configuration is readjusted so as to achieve a good match between input training data and their respective target for all pairs. The match is measured by way of a similarity measure, which can be formulated in terms of on objective function, or cost function. The aim is to adjust the parameters to incur low cost, that is, a good match.

More specifically, in the NN model, the input training data items are applied to the input layer (IL) and passed through a cascaded group(s) of convolutional layers CL1, CL2 and possibly one or more pooling layers PL1, PL2, and are finally passed to one or more fully connected layers. The convolutional module is responsible for feature based learning (e.g. identifying features in the patient characteristics and context data, etc.), while the fully connected layers are responsible for more abstract learning, for instance, the impact of the features on the treatment. The output layer OL includes the output data that represents the estimates for the respective targets.

The exact grouping and order of the layers as per Fig. 2 is but one exemplary embodiment, and other groupings and order of layers are also envisaged in different embodiments. Also, the number of layers of each type (that is, any one of CL, FL, PL) may differ from the arrangement shown in Fig. 2. The depth of the CNN may also differ from the one shown in Fig. 2 All that has been said above is of equal application to other NNs envisaged herein, such as fully connected classical perceptron type NN, deep or not, and recurrent NNs, or others.

The annotated (labelled) training data, as envisaged herein may need to be reformatted into structured form. As mentioned, the annotated training data may be arranged as vectors or matrices or tensor (arrays of dimension higher than 2). This reformatting may be done by a data pre-processor module (not shown), such as scripting program or filter that runs through patient records of the HIS of the current facility to pull up a set of patient characteristics.

The training data sets are applied to the initially configured CNN and is then processed according to a learning algorithm such as the FB-propagation algorithm as mentioned before.

At the end of the training phase, the so pre-trained CNN may then be used in deployment phase to compute the decision support information for new data, that is, newly acquired images not present in the training data. For example, as shown in Fig. 2, the newly acquired image 50 is a 2D image of the subject 106 acquired by the camera system 120 shown in Fig. 1. The output of the pretrained CNN is the geometry parameter of collimation, which may be a predicted size and position of the light field of the collimation with respect to the subject 106.

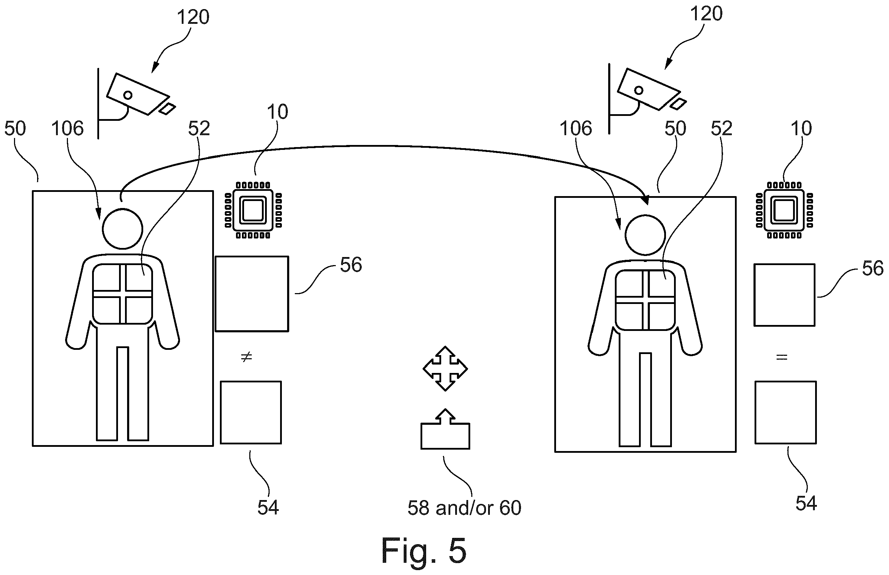

Fig. 5 illustrates an exemplary deployment phase. As shown in Fig. 5, the camera system 120 may capture a plurality of frames of scene containing the subject 106. The acquired image data 50 may show a current light field of the collimation that is projected onto the subject. The apparatus 10 determines the geometry parameter of the collimation based on the acquired image data 50 in accordance with the method described herein, and determines a geometry parameter of a target light field of the collimation 54. The apparatus 10 then determines a geometry parameter of the current light field of the collimation 56, and compares the geometry parameter of the current light field of the collimation 56 with the geometry parameter of the target light field of the collimation 54. The geometry parameter of the current light field of the collimation 56 and the geometry parameter of the target light field of the collimation 54 may include a size of the light field and a position of the light field with respect to the subject 106. If the deviation exceeds a threshold, the apparatus may generate a collimation adjustment guidance 58 indicative of the determined deviation and/or generate a control signal 60, which is used for controlling a collimator of the imaging system to adjust a collimator setting to reduce the deviation. In some examples, the collimation adjustment guidance may comprise a display showing the target light field of the collimation that is overlaid on the current light field of the collimation projected onto the subject. In some examples, the collimation adjustment guidance may comprise a visual instruction, such as LEDs on either side. In some further examples, the collimation adjustment guidance may comprise an audio command. In some examples, the control signal may comprise a control signal configured to control a motor to control a knob to adjust the collimator of the imaging system. In some examples, the control signal may comprise a control signal configured to inject a system command to control the collimator of the imaging system.

The above steps may be repeated and controlled via a feedback loop. For example, the apparatus 10 may continuously monitor the current light field of collimation that is projected onto the subject in accordance with the image data 50 acquired by the camera system 120, and determine the deviation between the geometry parameter of the current light field of the collimation and the geometry parameter of the target light field of the collimation. The collimation adjustment guidance 58 and/or the control signal 60 may be adapted continuously based on the deviation. For example, the apparatus 10 may continuously adapt the collimation adjustment guidance 56 to allow an operator to adapt the light field continuously until the geometry parameter of the current light field of the collimation matches the target geometry parameter of the light field of the collimation. In another example, the apparatus 10 may continuously generate a control signal 60 to fit one or more motors to control the knobs or handles to adjust the collimator while continuously segmenting the actual light field and controlling towards the target light field of collimation. In a further example, the apparatus 10 may continuously generate a control signal 60 to inject system commands to control the collimator while continuously segmenting the actual light field and controlling towards the target light field of collimation.

Some or all of the above-mentioned steps may be implemented in hardware, in software or in a combination thereof. Implementation in hardware may include a suitably programmed FPGA (field-programmable-gate-array) or a hardwired IC chip. For good responsiveness and high throughput, multi-core processors such as GPU or TPU or similar may be used to implement the above described training and deployment of the machine learning algorithm, in particular for NNs.

In another embodiment, the camera system 120 may comprise a 2D camera and another sensor, e.g., an IR and / or a depth sensor (e.g., time-of-flight sensor), as typically used in the three-dimensional cameras such as the Microsoft Kinect Azure camera. In this embodiment, the RGB data are used to detect the current light field of the collimation, whereas the data from any or all the other sensors are used to predict the optimal light field. This embodiment requires that the different sensor images of the camera system are registered spatially, which is typically the case. Hence, the parameters of the current light field, during training and inference, can be transformed from the RGB data to the data of the other sensors. The CNN (or other AI approach) can be trained using features of any of the sensor data.

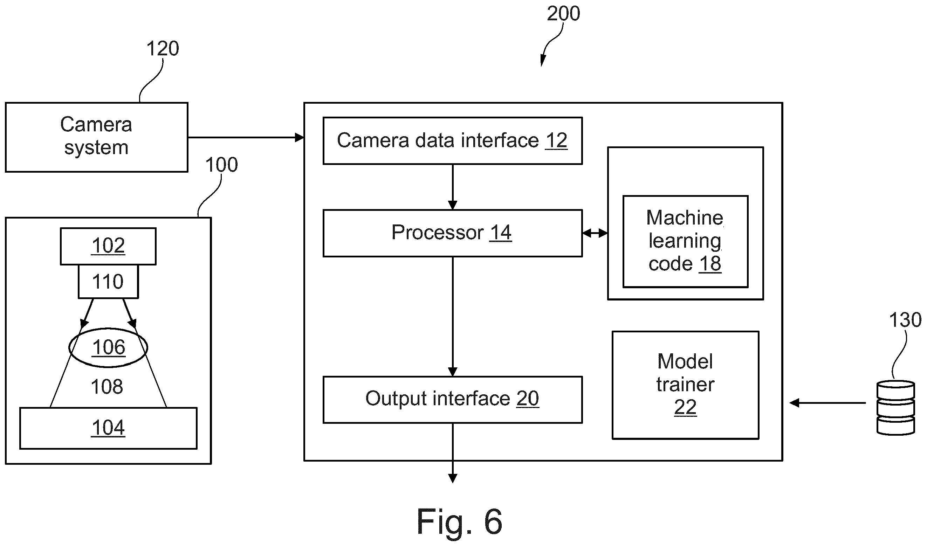

Fig. 6 illustrates a further exemplar apparatus 10. In this illustrated example, the apparatus 10 further comprises a model trainer 22. The model trainer 22 is provided to facilitate or otherwise support training of the machine-learning algorithm. Although not shown in Fig. 6, the model trainer 32 may include one or more processors, one or more memory elements, and other components to implement model training logic. The model trainer 22 may have an interface (not shown) that is configured to access the X-ray image data stored in a database 130. The database 130 may be a PACS system. In this embodiment, online-learning may be applied to train or re-train the machine-learning algorithm. The machine-learning model may be trained or retrained by with access to the X-ray images exported to the PACS and the corresponding meta-data on the collimation (e.g., the shutter-cropped region). In some examples, a spatial calibration and alignment of the 2D camera and the X-ray detector coordinate system may be required. The machine-learning model of the current collimation can be (re-)trained in online manner when assigning different levels of experience to the radiographers and using the collimation settings of experienced radiographers as labels, such as weak labels. If there is not system integration access to X-ray images can be achieved by means of (a) setting up a Digital Imaging and Communications in Medicine (DICOM) node, (b) sniffing the display signal, or (c) filming the control room monitor.

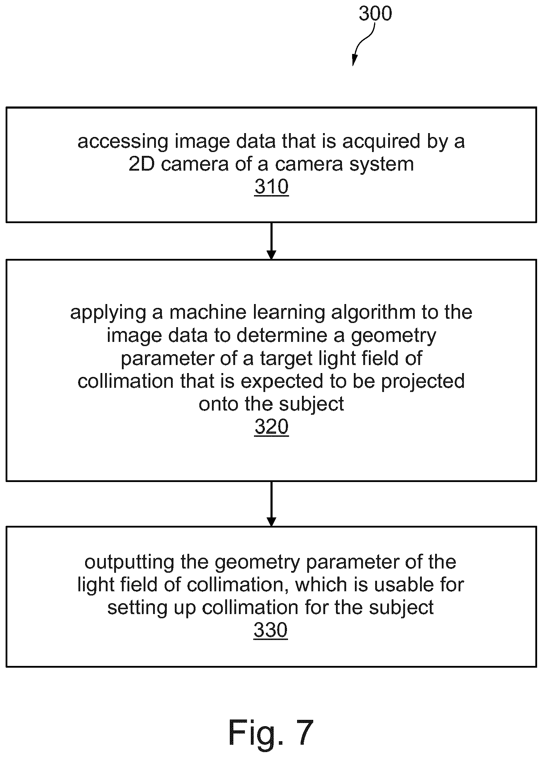

Further, in Fig. 7, an exemplary method is illustrated as a collection of blocks in a logical flow chart, which represents operations that may be implemented in hardware, software, or a combination thereof. The various operations are depicted in the blocks to illustrate the functions that may be performed. In the context of software, the blocks represent computer instructions that, when executed by one or more processing subsystems, may perform the recited operations.

The order in which the exemplary method is described is not intended to be construed as a limitation, and any number of the described blocks may be combined in any order to implement the exemplary method disclosed herein, or an equivalent alternative method. Additionally, certain blocks may be deleted from the exemplary method or augmented by additional blocks with added functionality without departing from the spirit and scope of the subject matter described herein. For discussion purposes, the exemplary method will be described with reference to the elements of Figs. 1-6.

At step 310, the method comprises the step of accessing image data that is acquired by a 2D of a camera system. The 2D camera has a field of view to capture a scene containing a subject and an X-ray detector, wherein the image data is obtained prior to the imaging examination of the subject. An exemplary camera system 120 is shown in Fig. 1, and exemplary image data 50 acquired by the exemplary camera system 120is show in Fig. 2 and 5.

At step 320, the method 300 further comprises the step of applying a machine learning algorithm to the image data to determine a geometry parameter of a target light field of collimation that is expected to be projected onto the subject. The machine learning algorithm has been trained using a training dataset comprising a plurality of training data examples. In some examples, the plurality of training examples may comprise a training data example comprising (i) image data of a scene comprising a subject with a light field of collimation projected onto said subject, wherein the image data was obtained during a previous imaging procedure for the same or another subject, and (ii) a geometry parameter of the collimation derived from the image data. An exemplary training pair is shown in Fig. 3. In some examples, the plurality of training examples may comprise a training data example comprising (i) an X-ray image that was acquired during a previous imaging procedure of the same or another subject, said X-ray image showing a subject's exterior, and (ii) a geometry parameter of the collimation derived from the X-ray image. An exemplary training pair is shown in Fig. 4.

In one option, the at least one training data example may further comprise non-image subject. The non-image subject data of the subject may comprise one or more of the following: a weight of the subject, an age of the subject, a gender of the subject, a quantification of a fitness level of the subject, a disease diagnosis associated with the subject, a medication record associated with the subject, and a vital parameter record associated with the subject. The set of instructions, when executed by the processor, cause the processor to use the non-image subject data as additional input to the machine learning algorithm.

At step 330, the method 300 further comprises the step of outputting the geometry parameter of the light field of collimation, which is usable for setting up collimation for the subject. For example, as shown in Fig. 2, the geometry parameter of the collimation is output from the machine learning algorithm.

In some implementations, the image data shows a current light field of the collimation that is projected onto the subject. The method 300 may further comprise the steps of determining a deviation between a geometry parameter of the current light field of the collimation and the geometry parameter of the target light field of the collimation, and if the deviation exceeds a threshold, generating a collimation adjustment guidance indicative of the determined deviation and/or generate a control signal, which is used for controlling a collimator of the imaging system to adjust a collimator setting to reduce the deviation. The above steps may be repeated until the deviation is less then the threshold. In some examples, the collimation adjustment guidance comprises one or more of the following: a display showing the target light field of the collimation that is overlaid on the current light field of the collimation projected onto the subject, a visual instruction, and an audio command. In some examples, the control signal comprises one or more of a control signal configured to control a motor to control a knob to adjust the collimator of the imaging system, and a control signal configured to inject a system command to control the collimator of the imaging system.

In another exemplary embodiment of the present invention, a computer program or a computer program element is provided that is characterized by being adapted to execute the method steps of the method according to one of the preceding aspects, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus (10) for determining a collimation configuration in an imaging system, the apparatus comprising:
- a camera data interface (12) configured to access image data that is acquired by a two-dimensional, 2D, camera of a camera system, wherein the 2D camera has a field of view to capture a scene containing a subject and an X-ray detector, wherein the image data is obtained prior to an imaging examination of the subject;
- a memory (16) comprising instruction data representing a set of instructions;
- a processor (14) configured to communicate with the camera data interface and the memory and to execute the set of instructions, wherein the set of instructions, when executed by the processor, cause the processor to apply a machine learning algorithm to the image data to determine a geometry parameter of a target light field of collimation that is expected to be projected onto the subject, wherein:
- the machine learning algorithm is represented by algorithm data which is stored in the memory and accessed by the processor, and
the machine learning algorithm has been trained using a training dataset comprising a plurality of training data examples, wherein the plurality of training examples comprises one or more of:
- a training data example comprising (i) image data of a scene comprising a subject with a light field of collimation projected onto said subject, wherein the image data was obtained during a previous imaging procedure for the same or another subject, and (ii) a geometry parameter of the collimation derived from the image data; and
- a training data example comprising (i) an X-ray image that was acquired during a previous imaging procedure of the same or another subject, said X-ray image showing a subject's exterior, and (ii) a geometry parameter of the collimation derived from the X-ray image; and
- an output interface (20) configured to output the geometry parameter of collimation.

2. The apparatus according to claim 1,
wherein the image data shows a current light field of the collimation that is projected onto the subject; and
wherein the set of instructions, when executed by the processor, cause the processor to:
- determine a deviation between a geometry parameter of the current light field of the collimation and the geometry parameter of the target light field of the collimation; and
- if the deviation exceeds a threshold, generate a collimation adjustment guidance indicative of the determined deviation and/or generate a control signal, which is used for controlling a collimator of the imaging system to adjust a collimator setting to reduce the deviation.

3. The apparatus according to claim 2,
wherein the set of instructions, when executed by the processor, cause the processor to:
- continuously monitor the current light field of collimation that is projected onto the subject, and determine the deviation between the geometry parameter of the current light field of the collimation and the geometry parameter of the target light field of the collimation, until the deviation is less than the threshold.

4. The apparatus according to claim 2 or 3,
wherein the collimation adjustment guidance comprises one or more of the following:
- a display showing the target light field of the collimation that is overlaid on the current light field of the collimation projected onto the subject;
- a visual instruction; and
- an audio command.

5. The apparatus according to any one of claims 2 to 4,
wherein the control signal comprises one or more of:
- a control signal configured to control a motor to control a knob to adjust the collimator of the imaging system; and
- a control signal configured to inject a system command to control the collimator of the imaging system.

6. The apparatus according to any one of the preceding claims,
wherein at least one training data example further comprises a score indicative of a collimation quality of an X-ray image acquired during the previous imaging procedure.

7. The apparatus according to claim 6,
wherein the score comprises a user input score, or a score generated based on an analysis of the X-ray image.

8. The apparatus according to any one of the preceding claims,
wherein the 2D camera comprises an RGB camera.

9. The apparatus according to any one of the preceding claims,
wherein the camera data interface is further configured to access image data acquired by a three-dimensional, 3D, camera of the camera system;
wherein at least one training data example further comprises image data acquired by a 3D camera during the previous imaging procedure; and
wherein the set of instructions, when executed by the processor, cause the processor to use the image data acquired by the 3D camera of the camera system as additional input to the machine learning algorithm.

10. The apparatus according to any one of the preceding claims, further comprising subject data interface configured to access the non-image subject data from an electronic health record of the subject;
wherein at least one training data example further comprises non-image subject data; and
wherein the set of instructions, when executed by the processor, cause the processor to use the non-image subject data as additional input to the machine learning algorithm.

11. The apparatus according to claim 10, wherein the non-image subject data of the subject comprises at least one of:
- a weight of the subject;
- an age of the subject;
- a gender of the subject;
- a quantification of a fitness level of the subject;
- a disease diagnosis associated with the subject;
- a medication record associated with the subject; and
- a vital parameter record associated with the subject

12. The apparatus according to any one of the preceding claims,
wherein the geometry parameter of the target light field of collimation comprises a size of the light field of the collimation and a position of the light field of the collimation with respect to the subject.

13. The apparatus according to any one of the preceding claims,
wherein the machine learning algorithm comprises at least one of:
- a convolutional neural network, CNN;
- a transformer;
- a generalized Hough transform; and
- an edge detection algorithm.

14. A method for determining a collimation configuration in an imaging system, the method comprising:
- accessing (310) image data that is acquired by a two-dimensional (2D) camera of a camera system, wherein the 2D camera has a field of view to capture a scene containing a subject, wherein the image data is obtained prior to the imaging examination of the subject;
- applying (320) a machine learning algorithm to the image data to determine a geometry parameter of a target light field of collimation that is expected to be projected onto the subject, wherein:
the machine learning algorithm has been trained using a training dataset comprising a plurality of training data examples, wherein the plurality of training examples comprises one or more of:
- a training data example comprising (i) image data of a scene comprising a subject with a light field of collimation projected onto said subject, wherein the image data was obtained during a previous imaging procedure for the same or another subject, and (ii) a geometry parameter of the collimation derived from the image data; and
- a training data example comprising (i) an X-ray image that was acquired during a previous imaging procedure of the same or another subject, said X-ray image showing a subject's exterior, and (ii) a geometry parameter of the collimation derived from the X-ray image; and
- outputting the geometry parameter of the light field of collimation, which is usable for setting up collimation for the subject.

15. A computer readable medium comprising transitory or non-transitory data representing instructions arranged to cause a processor system to perform the method according to claim 14.
